# EUROPÄISCHE PATENTSCHRIFT

(11) **EP 2 513 126 B1**
(45) Veröffentlichungstag und Bekanntmachung des Hinweises auf die Patenterteilung: **29.07.2015**
(21) Anmeldenummer: 10792885.5
(22) Anmeldetag: 09.12.2010
(51) Int. Cl.: C07F 15/00, C07F 1/08, C07C 68/00, C08G 64/36

(54) **BIMETALLISCHE KOMPLEXE UND IHRE VERWENDUNG IN DER DIARYLCARBONATHERSTELLUNG**
BIMETALLIC COMPLEXES AND THEIR USE IN THE PREPARATION OF DIARYLCARBONATE
COMPLEXES BIMETALLIQUES ET LEUR UTILISATION POUR LE PREPARATION DU CARBONATES DIARYLENE

(30) Priorität: 14.12.2009 DE 102009058053
(43) Veröffentlichungstag der Anmeldung: 24.10.2012
(73) Patentinhaber: Bayer Intellectual Property GmbH, 40789 Monheim (DE)
(72) Erfinder: GÜRTLER, Christoph, 50676 Köln (DE); MÜLLER, Thomas Ernst, 81827 München (DE); OOMS, Pieter, 47800 Krefeld (DE); RECHNER, Johann, 47906 Kempen (DE); RISSE, Friedhelm, 50739 Köln (DE); PROKOFIEVA, Angelina, 52062 Aachen (DE); DORO, Franco, 52062 Aachen (DE); KÖHLER, Burkhard, 34289 Zierenberg (DE); LEITNER, Walter, 52074 Aachen (DE); WOLF, Aurel, 42489 Wülfrath (DE)
(74) Vertreter: BIP Patents
(86) Internationale Anmeldenummer: PCT/EP2010/069323
(87) Internationale Veröffentlichungsnummer: WO 2011/073087

(56) Entgegenhaltungen:
- WO-A1-2008/054024
- DE-A1- 2 738 437

## Beschreibung

Die Erfindung betrifft bimetallische Komplexe, wobei der Ligand eine Salophen-Einheit enthält, die Kupfer, Mangan oder Kobalt komplexiert und eine Phenanthrolin-Einheit, die Palladium komplexiert und beide Systeme durch ein durchkonjugiertes System verknüpft werden. Ferner betrifft die Erfindung die Verwendung dieser bimetallischen Komplexe als Katalysatoren für die oxidative Carbonylierung von aromatischen Hydroxyverbindungen zu Diarylcarbonaten sowie ein Verfahren zur Herstellung von Diarylcarbonaten mithilfe des bimetallischen Komplexes als Katalysator.

Diarylcarbonate eignen sich zur Herstellung von Polycarbonaten nach dem Schmelzeumesterungsverfahren (siehe z.B. in Chemistry and Physics of Polycarbonates, Polymer Reviews, H. Schnell, Vol. 9, John Wiley and Sons, Inc., 1964), zur Herstellung von Phenylurethanen oder sind Vorprodukte von Wirkstoffen aus dem Pharma- und Pflanzenschutzsektor.

Es ist bekannt, dass Diarylcarbonate durch Phasengrenzflächenphosgenierung (Schotten-Baumann-Reaktion) von aromatischen Hydroxyverbindungen gewonnen werden können. Dieses Verfahren hat aber erhebliche Nachteile wie den Einsatz von Phosgen und Lösungsmitteln, wie Methylenchlorid.

Diarylcarbonate können auch über oxidative Direktcarbonylierung von aromatischen Hydroxyver-bindungen in Gegenwart von CO, O₂ und eines Edelmetall-Katalysators hergestellt (siehe z.B. DE-OS 27 38 437, US-A 4,349,485, US-A 5,231,210, EP-A 667 336, EP-A 858 991, US-A 5,760,272). Als Edelmetall wird bevorzugt Palladium eingesetzt. Zusätzlich können ein Cokatalysator (z.B. Mangan- oder Kobaltsalze), eine Base, Bromidquellen, quaternäre Salze, verschiedene Chinone bzw. Hydrochinone und Trockenmittel eingesetzt werden. Dabei kann in einem Lösungsmittel wie Chlorbenzol gearbeitet werden.

Im bekannten Stand der Technik wie z.B. J. Mol. Cat. A: Chem. 2000, 151, 37-45 werden Kupfer-, Mangan- oder Kobaltsalze im Überschuss relativ zu dem Edelmetall-Katalysator eingesetzt, wodurch der Katalysator und der Cokatalysator daher nur mit geringer Effizienz genutzt werden kann.

Zudem weisen die bekannten Verfahren keine genügende Katalysatorstandzeit auf. Insbesondere kommt es zur Abscheidung von metallischem Palladium.

Ausgehend vom bekannten Stand der Technik bestand daher Bedarf nach Katalysatoren, die eine Reaktionsführung ohne Einsatz zusätzlicher Mengen an Kupfer-, Mangan- oder Kobaltsalzen als Cokatalysator und eine gesteigerte Effizienz des Katalysators (z.B. Turn Over Number, TON) ermöglichen. Zudem bestand Bedarf danach, Katalysatoren bereitzustellen, welche eine höhere Stabilität aufweisen.

Das zu lösende Problem wurde überraschenderweise durch einen bimetallischen Komplex, in dem das zweite Metall stöchiometrisch relativ zu Palladium im Komplex eingebaut ist, gelöst. Diese Verbindung führt dazu, dass der Cokatalysator in synergistischer Weise genutzt werden kann und die Standzeit des bimetallischen Katalysators relativ zum Vergleichssystem erhöht ist.

Es wurden bimetallische Katalysatoren gefunden, in denen das katalytisch aktive Palladium und der redoxaktive Cokatalysator in einem Molekül kombiniert sind und dadurch eine beschleunigte Reoxidation des Palladiums ermöglichen, die zu einem synergistischen Effekt führen.

Bimetallische Komplexe der Formel (1) sind in WO 2008/054024 als Photosensibilisatoren für photovoltaische Zellen beschrieben: 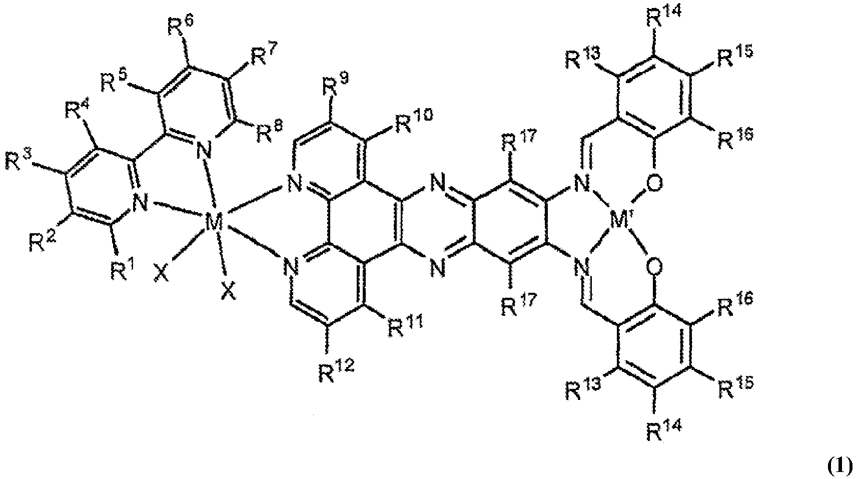 wobei M = Ru, Os, Fe, Re, Rh und M' = Ni, Co, Zn, Mn, Pt, Pd. Das an die Phenanthrolin-Einheit gebundene Metall wird zusätzlich zwingend durch weitere Bipyridin-Liganden komplexiert. Als Katalysatoren für oxidative Carbonylierungen, wie die Herstellung von Diphenylcarbonat aus Phenol, Kohlenmonoxid und Sauerstoff, sind diese Komplexe nicht geeignet, da durch die Blockierung der Koordinationsstelle am Metallzentrum M durch die Bipyridineinheit die Koordinationsstellen nicht mehr für die Anlagerung von Reaktanden zur Verfügung stehen. Als katalytisch aktives Metall für oxidative Carbonylierungen (Metallzentrum M) ist Palladium bevorzugt, das sich in einer Koordinationssphäre bestehend aus zwei *cis*-ständigen und zwei schwach koordinierten Liganden befindet (J. Mol. Cat. A: Chem. 2000, 151, 37-45).

Die Synthese solcher Liganden und Komplexe ist ferner beschrieben in European Journal of Inorganic Chemistry 2003, 10, 1900-1910. Dabei werden zwei Strategien angewandt, der Aufbau beginnend von der Phenanthrolin-Einheit und Komplexierung erst nach der kompletten Synthese des Liganden und der Aufbau beginnend von der Salophen-Einheit, wobei ein Templatverfahren gewählt wird, bei dem die Synthese in Gegenwart des Zentralions, Ni(II), vorgenommen wird. Hierfür wird als Zwischenprodukt der 3,4-Dinitro-Salophen-Ni(II)-Komplex benötigt, Formel (2), dessen Synthese in Dalton Transactions 2009, 10, 1792-1800 und der dort zitierten Literatur beschrieben wird.

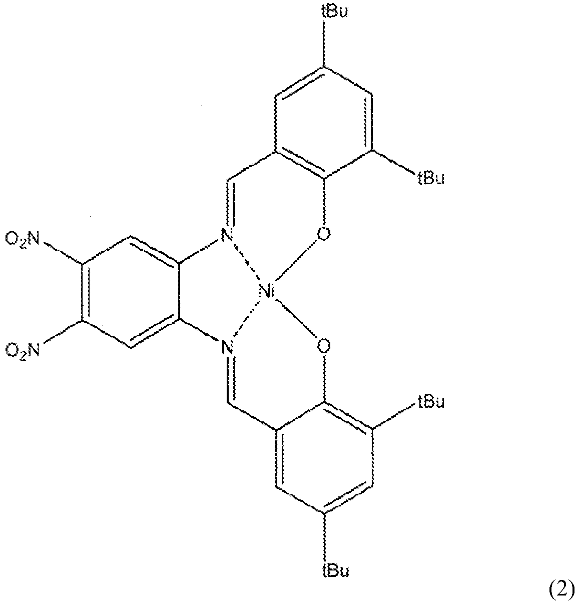

Für bestimmte Anwendungen, insbesondere in der Katalyse, sind Komplexe erwünscht, deren Metallionen in der Phenanthrolin-Einheit nicht durch weitere Bipyridin-Liganden komplexiert sind. Ferner ist der Templat-Weg einfacher durchführbar, aber für Kupfer als Zentralatom noch nicht beschrieben.

Es wurde nun gefunden, das sich aus 3,4-Dinitro-Salophen-Cu(II)-Komplexen Liganden herstellen lassen, die zusätzlich eine Phenanthrolin-Einheit enthalten, die mit Palladiumhalogenid-Komplexen zu bimetallischen Komplexen umgesetzt werden können, bei denen das Palladium nicht durch weitere Bipyridin-Liganden stabilisiert ist. Dabei war zu erwarten, dass das Palladium durch zwei Phenanthrolin-Liganden koordiniert wird, da die beiden einzähnigen Liganden Hal leichter zu substituieren sind, als Bipyridinchelate. Die Bildung von Palladiumbisphenanthrolin-Komplexen trat jedoch nicht ein.

Gegenstand der Erfindung sind daher Komplexe der allgemeinen Formel (3), 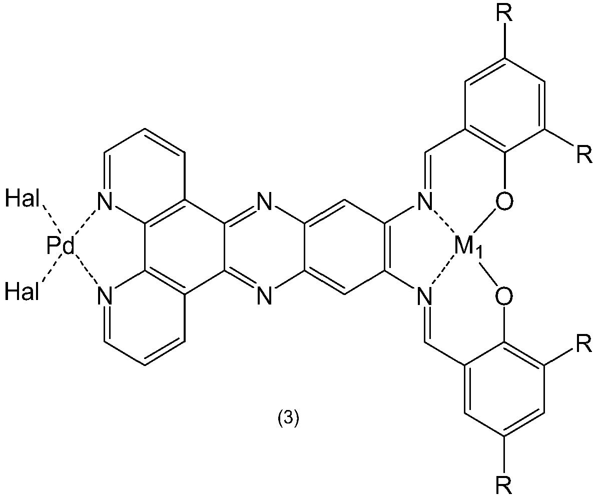 wobei
- R: für Wasserstoff, Fluor, Chlor, eine Nitrogruppe oder einen C₁ -C₂₂-Alkylrest, vorzugsweise für Methyl, Isopropyl oder *tertiär*-Butyl, ganz besonders bevorzugt für *tertiär*-Butyl, oder einen C₆-C₂₂-Arylrest, vorzugsweise für Phenyl, Tolyl, 2-(1,4-Dihydroxy)phenyl
- Hal: für Chlorid, Bromid oder Iodid, vorzugsweise für Chlorid oder Bromid, ganz besonders bevorzugt Bromid, oder ein Alkoholat, beispielsweise Phenolat oder Methylat oder ein schwach koordinierendes Anion, wie Triflat, Tosylat, Mesylat, Tetrafluoroborat, Perchlorat und Hexafluorophosphat und
- M₁: für Kupfer, Mangan oder Kobalt steht.

Ein weiterer Gegenstand der Erfindung sind Komplexe der allgemeinen Formel 4a und 4b, die als Zwischenprodukte bei der Herstellung des erfindungsgemäßen bimetallischen Komplexes auftreten. 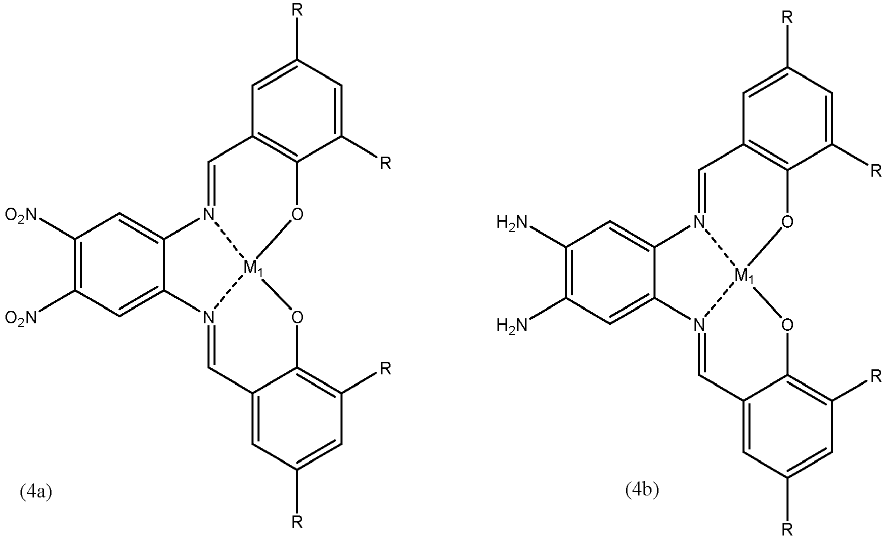 wobei R und M₁ die oben angegebene Bedeutung haben.

Gegenstand der Erfindung ist auch die Verwendung der bimetallischen Komplexe der Formel 3 als Katalysatoren für die oxidative Carbonylierung von aromatischen Hydroxyverbindungen zu Diarylcarbonaten.

Die Synthese von Verbindungen der Formel 3 erfolgt durch Hydrierung der Nitrogruppen der Verbindung der Formel 4a zu Verbindungen der Formel 4b, die anschließende Umsetzung mit Phendion der Formel 5 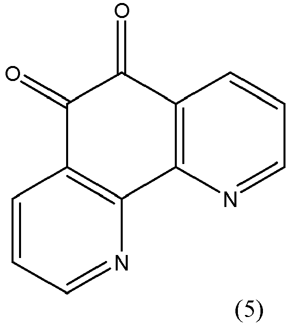 zu Liganden der Formel 6 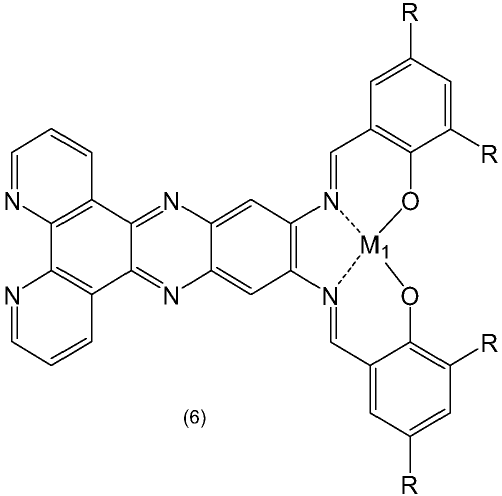 und Komplexierung mit einer Verbindung der Formel 7,

PdHal₂L₂ (7)

wobei Hal die oben angegebene Bedeutung hat und L für einen optional vorhandenen Liganden, wie z.B. Acetonitril, Triarylphosphine, Cyclooctadien, oder Norbornadien, steht.

Das zur Herstellung der Komplexe nach Formel 4a benötigte 4,5-Dinitro-o-phenylendiamin kann z.B. nach dem Literturzitat Cheeseman G.W.H. J. Chem.Soc., 1962, 1170 hergestellt werden. Die Hydrierung zu Verbindungen der Formel 4b erfolgt durch Hydrierung der Verbindungen der Formel 4a mit wasserstoffabgebenden Reagenzien, wie H₂, Hydrazin, Cyclohexen oder Ameisensäure in Gegenwart von Katalysatoren aus den Metallen Ni, Co, Pd, Pt, Ru oder Rh, die auf Trägern, wie Kohle oder anorganischen Substanzen, immobilisiert sein können.

Die Umsetzung mit Phendion erfolgt z.B. durch gemeinsames Erhitzen unter Rückfluss mit äquimolaren Mengen an Verbindungen der Formel 4b in Lösungsmitteln, wie Methanol, Ethanol oder Isopropanol, Chloroform, Benzol, Toluol oder deren Gemischen, wobei optional das gebildete Wasser durch azeotrope Destillation entfernt werden kann.

Die Bildung der Komplexe der Formel 3 erfolgt durch Umsetzung der Liganden der Formel 6 mit Verbindungen der Formel 7 in Lösungsmitteln, wie Methylenchlorid, Chloroform, 1,2-Dichlorethan, Benzol, Chlorbenzol, DMF, Diethylether, Methyl-tert-butylether oder THF bei Temperaturen von 10°C bis zum Siedepunkt der Lösungsmittel unter Normaldruck.

Die erfindungsgemäßen bimetallischen Komplexe der Formel 3 eignen sich zur Katalyse der oxidativen Carbonylierung von aromatischen Hydroxyverbindungen zu Diarylcarbonaten.

Aromatische Hydroxyverbindungen für das erfindungsgemäße Verfahren sind solche der Formel 8

Ar-OH (8),

worin
- Ar: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C₁-C₄-Alkyl, geradkettiges oder verzweigtes C₁-C₄-Alkoxy, geradkettiges oder verzweigtes C₁-C₄-Alkoxycarbonyl, die mit Phenyl, Cyano und Halogen (z.B. F, Cl, Br) substituiert sein können und wobei weiterhin die heterocyclischen Reste mit einem ankondensierten Benzolkern verbunden sein können.

Beispiele für aromatische Hydroxyverbindungen der Formel 8 sind: Phenol, *o*-, *m-* und *p*-Kresol, *o*-, *m*- und *p*-Isopropylphenol, die entsprechenden Halogen- bzw. Alkoxyphenole, wie *p*-Chlorphenol bzw. *p*-Methoxyphenol, Salicylsäuremethylester, Salicylsäureethylester, ferner Monohydroxyverbindungen des Naphthalins, Anthracens und Phenanthrens, weiterhin 4-Hydroxypyridin und Hydroxychinoline. Vorzugsweise werden Phenol und gegebenenfalls substituierte Phenole, ganz besonders bevorzugt Phenol eingesetzt.

Die Herstellung eines Diarylcarbonats über oxidative Carbonylierung erfolgt durch Umsetzung einer aromatischen Hydroxyverbindung mit Kohlenmonoxid und Sauerstoff in Gegenwart eines erfindungsgemäßen Katalysators der Formel 3.

Die oxidative Carbonylierung von aromatischen Hydroxyverbindungen mit Hilfe der erfindungsgemäßen Katalysatoren nach Formel 3 erfolgt in flüssiger Phase in Gegenwart oder Abwesenheit von Lösungsmitteln, wie Chlorbenzol, Dichlorbenzol, Toluol, Xylol, DMF, Dimethylacetamid, Tetrahydrofuran, NMP, Ethylencarbonat oder Propylencarbonat bei Temperaturen von 25 bis 150°C, vorzugsweise von 60 bis 110°C in Gegenwart von Kohlenmonoxid-Sauerstoff Gemischen im Partialdruck-Verhältnis 1:1 bis 99:1, vorzugsweise 98:2 bis 60:40, gegebenenfalls in Gegenwart weiterer Inertgase, wie Stickstoff, Argon oder Kohlendioxid, wobei das Partialdruckverhältnis Inertgase zu Aktivgase 98:2 bis 2:98, vorzugsweise 95:5 bis 50:50 betragen kann und wobei der Gesamtdruck 1 bar bis 100 bar, vorzugsweise 5 bar bis 20 bar, beträgt.

Ferner können andere redoxaktive Substanzen, wie Chinone, Alkali- oder Erdalkaliiodide, Mangan-, Kobalt- oder Kupferverbindungen, in einem Gewichtsverhältnis Palladiumverbindung zu redoxaktiver Substanz von 1:1 bis 1:100, vorzugsweise 1:2 bis 1:20, anwesend sein. Beispiele für Chinone sind Benzochinon, Naphthochinon und Anthrachinon, sowie deren Substitutionsprodukte. Beispiele für Mangan-, Kobalt- oder Kupferverbindungen sind deren Oxide, Halogenide, Acetylacetonate oder Carboxylate.

Als weitere Cokatalysatoren oder Hilfsmittel können Oniumsalze, wie Ammonium-, Phosphonium- oder Sulfoniumsalze, Bleiverbindungen, wie Bleialkyle oder -oxide, Polymere, wie Polyvinylpyrrolidon, Alkylhalogenide, wie Dibromethan, in einem Gewichtsverhältnis von Palladiumverbindung zu Cokatalysator oder Hilfsmittel von 1:1 bis 1:200, vorzugsweise 1:10 bis 1:50, eingesetzt werden. Besonders bevorzugt werden die Bromide der Ammonium- oder Phosphoniumverbindungen in einem Gewichtsverhältnis von Palladiumverbindung zu Hilfsmittel von 1:1 bis 1:200, vorzugsweise 1:10 bis 1:50, eingesetzt.

Die Diarylcarbonate, hergestellt mit den erfindungsgemäßen Komplexen der Formel 3 als Katalysatoren, können zur Herstellung von Polycarbonaten oder Isocyanaten verwendet werden.

Die nachfolgenden Beispiele sollen die Erfindung illustrieren ohne sie jedoch einzuschränken.

### Beispiele

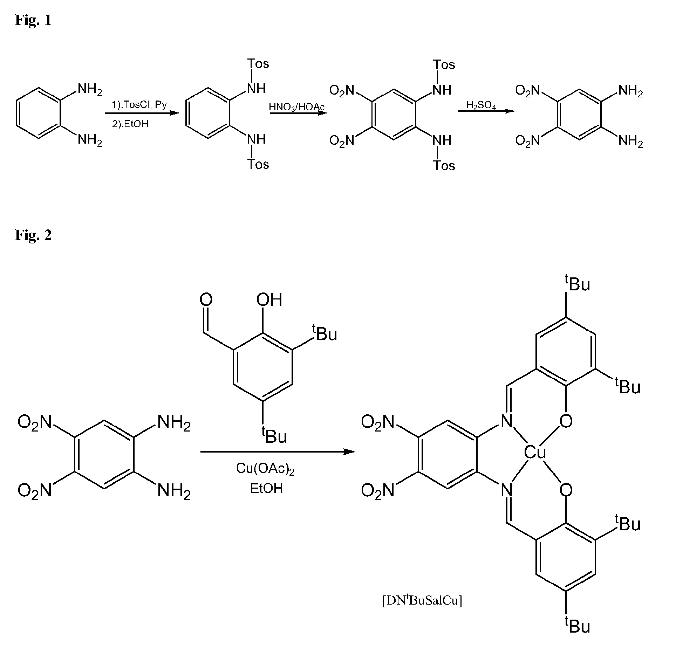

4,5-Dinitro-o-phenylenediamin wurde hergestellt nach Fig. 1 entsprechend dem Literaturzitat Cheeseman G. W. H. J. Chem. Soc., 1962, 1170.

### Beispiel 1

### Herstellung von [DN^{t}BuSalCu] (erfindungsgemäß entsprechend Formel 4a) nach Fig. 2.

Eine Lösung von 0,69 g (3,48 mmol) Cu(OAc)₂H₂0 in 20 mL Ethanol wurde zu einer Mischung von 0,68 g (3,48 mmol) 4,5-Dinitro-o-phenylenediamin und 1,63 g (6,96 mmol) 3,5-Di-tert-butylsalicalaldehyd in 80 mL Ethanol gegeben. Es wurde 80 min unter Rückfluss gekocht. Anschließend wurde der Niederschlag abfiltriert, mit n-Hexan gewaschen und im Vakuum getrocknet.
Ausbeute: 1,27 g (50 %).
Felddesorptions-Massenspektroskopie (FD-MS, D+Toluene) m/z (%): 691 (100) (C₃₆H₄₄CuN₄O₆)

### Beispiel 2

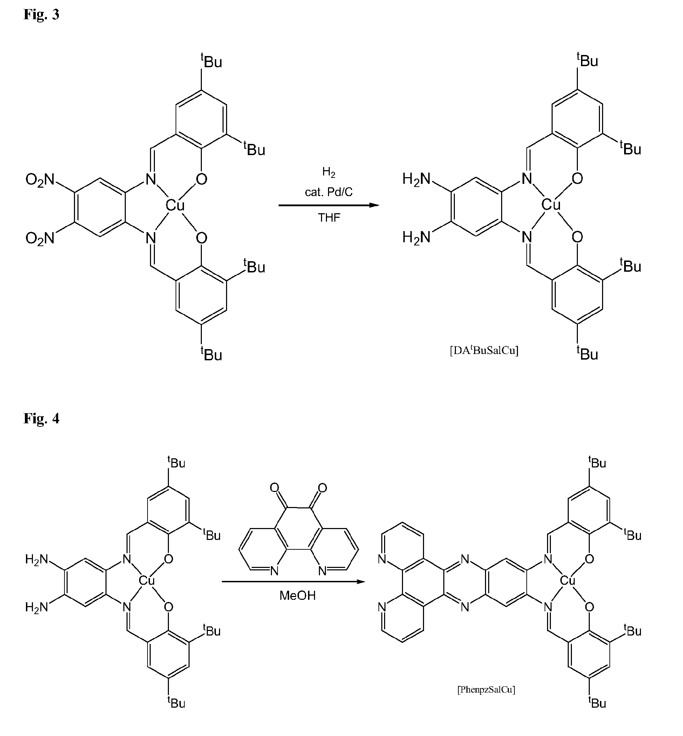

### Herstellung von [DA^{t}BuSalCu] (erfindungsgemäß entsprechend Formel 4b) nach Fig. 3.

Eine Suspension von 1,27 g (1,83 mmol) [DN^{t}BuSalCu] und 0,2 g Pd/C (10%) in 100 mL THF wurde in einem 250 mL Autoklav mit einem Wasserstoffdruck von 40 bar beaufschlagt, 12 h bei Raumtemperatur gerührt und anschließend filtriert. Das Filtrat wurde im Vakuum eingeengt, der Rückstand mit Hexan gewaschen und anschließend im Vakuum getrocknet.
Ausbeute: 1,16 g (100 %)
FD-MS (D+Toluene) m/z (%): 631 (100) (C₃₆H₄₈CuN₄O₂)

### Beispiel 3

### Herstellung von [PhenpzSalCu] (erfindungsgemäß entsprechend Formel 6) nach Fig. 4.

Eine Suspension von 0,5 g (0,79 mmol) [DA^{t}BuSalCu] in 30 mL Methanol wurde zu einer Lösung von 0,16 g (0,79 mmol) Phendion in 20 mL Methanol getropft. Dann wurde 2 h unter Rückfluss gekocht, wobei sich ein roter Niederschlag bildete, der abfiltriert, mit Petrolether gewaschen und im Vakuum getrocknet wurde.
Ausbeute: 0,5 g (78 %)
FD-MS (D+Toluene) m/z (%): 805 (100) (C₄₈H₅₀CuN₆O₂)

### Beispiel 4

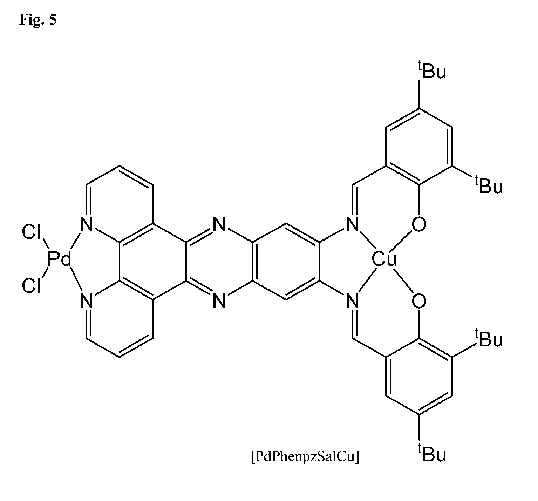

### Herstellung von [PdPhenpzSalCu] (erfindungsgemäß entsprechend Formel 1) nach Fig. 5.

Zu einer Lösung von 0,05 g (0,06 mmol) [PhenpzSalCu] in 5 mL Methylenchlorid wurde die Lösung von 0,01 g (0,06 mmol) [Pd(CH₃CN)₂Cl₂] gegeben und bei Raumtemperatur 16 h gerührt. Die flüchtigen Bestandteile wurden im Vakuum abgezogen und der Rückstand mit Hexan versetzt. Dann wurde das ausgefallene Produkt abfiltriert und im Vakuum getrocknet.
Ausbeute: 0,025 g (42 %)
FD-MS (D+Toluene) m/z (%): 981 (10) (C₄₈H₅₀Cl₂CuN₆O₂Pd), 805 (100) (C₄₈H₅₀CuN₆O₂)

### Beispiel 5

### Verwendung von [PdPhenpzSalCu] als Katalysator zur Herstellung von Diphenylcarbonat

Eine Mischung aus 894 mg (9,5 mmol) Phenol, 8,5 mg (0,0086 mmol) [PdPhenpzSalCu], 32 mg (0,29 mmol) Benzochinon, 222 mg (0.69 mmol) Tetrabutylammoniumbromid und 110 mg Molekularsieb (3 Å) wurde dreimal mit 10 bar einer Mischung aus 97% CO und 3% O₂ gespült und dann mit 18 bar dieser Gasmischung beaufschlagt. Dann wurde unter Rühren mit 900 rpm auf 90°C erhitzt. Die Analyse des Reaktionsgemisches mit Gaschromatographie nach einer Reaktionszeit von 3 h ergab eine Ausbeute von 5,1 mg (0,024 mmol) Diphenylcarbonat entsprechend einer TON des Katalysators bezogen auf Palladium von 2,8 und bezogen auf Kupfer von 2,8.

### Beispiel 6

Eine Mischung aus 954 mg (10,1 mmol) Phenol, 9,1 mg (0,0093 mmol) [PdPhenpzSalCu], 43,8 mg (0,39 mmol) Benzochinon, 212 mg (0.66 mmol) Tetrabutylammoniumbromid und 110 mg Molekularsieb (3 Å) wurde dreimal mit 10 bar einer Mischung aus 97% CO und 3% O₂ gespült und dann mit 18 bar dieser Gasmischung beaufschlagt. Dann wurde unter Rühren mit 900 rpm auf 90°C erhitzt. Die Analyse des Reaktionsgemisches mit Gaschromatographie nach einer Reaktionszeit von 14 h ergab eine Ausbeute von 5,8 mg (0,027 mmol) Diphenylcarbonat entsprechend einer TON des Katalysators bezogen auf Palladium von 3,0 und bezogen auf Kupfer von 3,0.

Die TON bezogen auf Palladium und Kupfer steigen mit der Reaktionszeit bei Verwendung der erfindungsgemäßen Verbindung.

### Vergleichsbeispiel 7

Nachstellung in Anlehnung an Literaturstelle J. Mol. Cat. A: Chem. 2000, 151, 37-45.

Eine Mischung aus 920 mg (9,78 mmol) Phenol, 4,6 mg (0,012 mmol) [Pd(Phen)Cl₂], 20 mg (0,056 mmol) [Co(acac)₃], 31,8 mg (0,29 mmol) Benzochinon, 219 mg (0,66 mmol) Tetrabutylammoniumbromid und 110 mg Molekularsieb (3 Å) wurde dreimal mit 10 bar einer Mischung aus 97% CO und 3% O₂ gespült und dann mit 18 bar dieser Gasmischung beaufschlagt. Dann wurde unter Rühren mit 900 rpm auf 90°C erhitzt. Die Analyse des Reaktionsgemisches mit Gaschromatographie nach einer Reaktionszeit von 3 h ergab eine Ausbeute von 5,8 mg (0,027 mmol) Diphenylcarbonat entsprechend einer TON des Katalysators bezogen auf Palladium von 3,4 und bezogen auf Kobalt von 0,5.

### Vergleichsbeispiel 8

Eine Mischung aus 994 mg (10,6 mmol) Phenol, 4,3 mg (0,011 mmol) [Pd(Phen)Cl₂], 18,2 mg (0,050 mmol) [Co(acac)₃], 36,2 mg (0,33 mmol) Benzochinon, 203 mg (0,60 mmol) Tetrabutylammoniumbromid und 110 mg Molekularsieb (3 Å) wurde dreimal mit 10 bar einer Mischung aus 97% CO und 3% O₂ gespült und dann mit 18 bar dieser Gasmischung beaufschlagt. Dann wurde unter Rühren mit 900 rpm auf 90°C erhitzt. Die Analyse des Reaktionsgemisches mit Gaschromatographie nach einer Reaktionszeit von 14 h ergab eine Ausbeute von 4,7 mg (0,022 mmol) Diphenylcarbonat entsprechend einer TON des Katalysators bezogen auf Palladium von 2,7 und bezogen auf Kobalt von 0,4.

In den Vergleichsbeispielen ist neben dem Palladiumkatalysator der Zusatz eines Kobaltsalzes als Cokatalysator notwendig. Das molare Verhältnis Palladium zu Cokatalysator beträgt 1:6,5, während bei Einsatz des bimetallischen Komplexes nach Beispielen 5 und 6 ein Verhältnis von 1:1 vorliegt.

**Tabelle 1**

| | Beispiel 5 | Beispiel 6 | Vergleichsbeispiel 7 | Vergleichsbeispiel 8 |
|---|---|---|---|---|
| TON(Pd) | 2,8 (3h) | 3,0 (14h) | 3,4 (3h) | 2,7 (14h) |
| TON(Cu) | 2,8 (3h) | 3,0 (14h) | - | - |
| TON(Co) | - | - | 0,5 (3h) | 0,4 (14h) |

Die TON bezogen auf Palladium und Kobalt sinken in den Vergleichsbeispielen bei verlängerter Reaktionszeit. Eine Gegenüberstellung des bimetallischen Katalysators nach Beispiel 5 und den entsprechenden Vergleichsbeispielen 7 und 8 belegt eine höhere Stabilität des erfindungsgemäßen bimetallischen Katalysators im Vergleich zu Katalysatoren aus dem Stand der Technik.

Die TON bezogen auf den Cokatalysator ist in den Vergleichsbeispielen 7 und 8 geringer als beim Einsatz des erfindungsgemäßen bimetallischen Katalysators nach Beispielen 5 und 6.

## Patentansprüche

1. Verbindung der Formel (3) 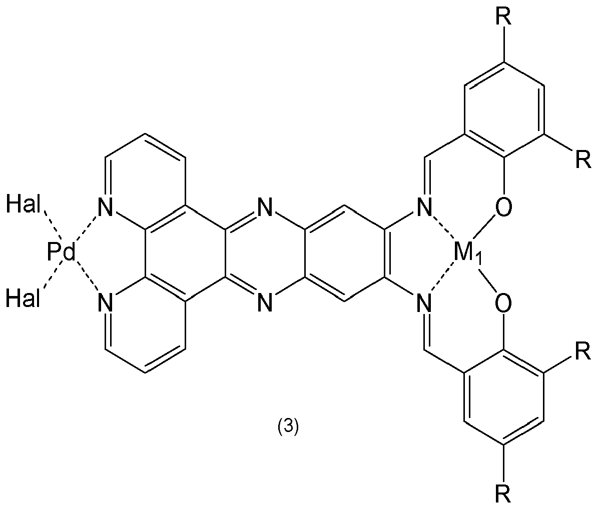 wobei
R für Wasserstoff, Fluor, Chlor, eine Nitrogruppe, einen C₁-C₂₂-Alkylrest, oder einen C₆-C₂₂-Arylrest
Hal für Chlorid, Bromid oder Iodid, ein Alkoholat, oder ein schwach koordinierendes Anion, und
M₁ für Kupfer, Mangan oder Kobalt steht.

2. Verfahren zur Herstellung der Verbindungen der Formel 3 durch Hydrierung der Nitrogruppen der Verbindung der Formel 4a zu Verbindungen der Formel 4b, die anschließende Umsetzung mit Phendion der Formel 5 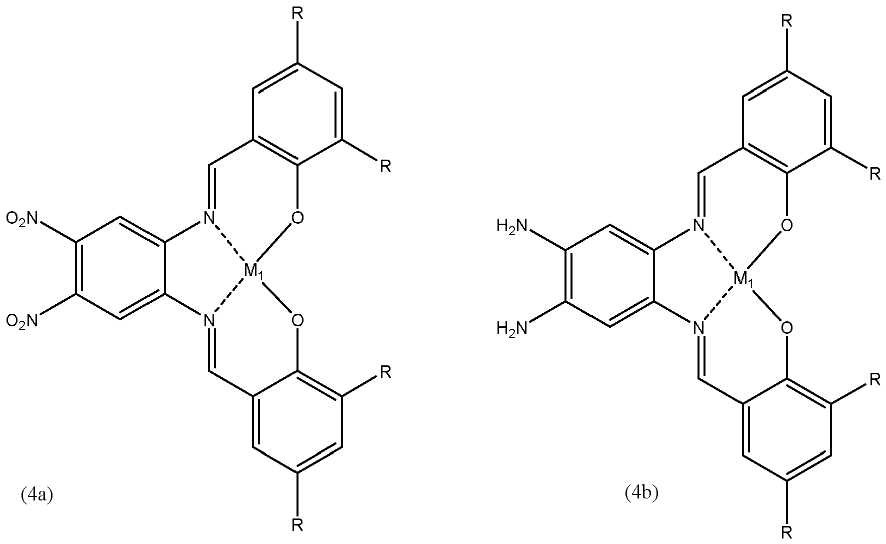 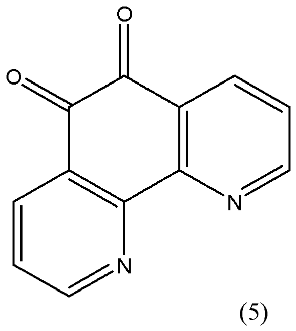 zu Liganden der Formel 6 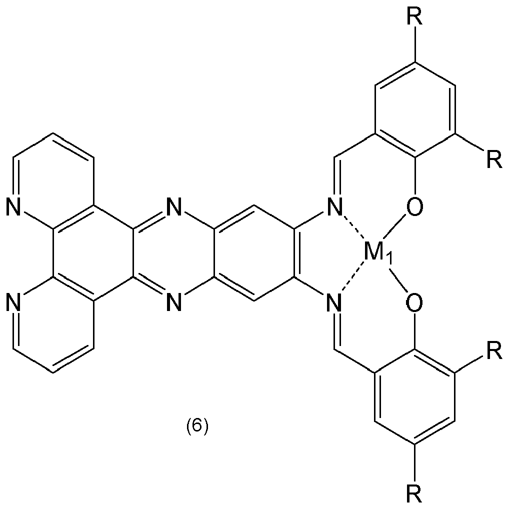 und Komplexierung mit einer Verbindung der Formel 7,
PdHal₂L₂ (7)
wobei Hal die oben angegebene Bedeutung hat und L für einen optional vorhandenen Liganden, steht.

3. Verbindungen der allgemeinen Formel 4a und 4b, 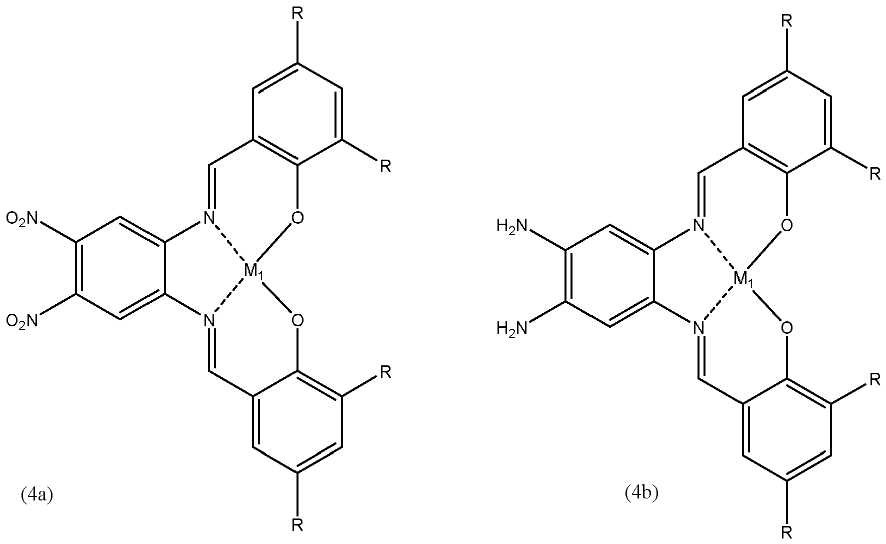 wobei R und M₁ die oben angegebene Bedeutung haben.

4. Verwendung einer oder mehrerer bimetallischen Verbindung(en) der Formel 3 gemäß Anspruch 1 als Katalysator.

5. Verwendung einer oder mehrerer bimetallischen Verbindung(en) der Formel 3 gemäß Anspruch 1 für die oxidative Carbonylierung von aromatischen Hydroxyverbindungen zu Diarylcarbonaten

6. Verfahren zur Herstellung von Diarylcarbonaten, **dadurch gekennzeichnet, dass** eine aromatische Hydroxyverbindung mit Kohlenmonoxid und Sauerstoff in Gegenwart des Katalysators der Formel 3 gemäß Anspruch 1 umgesetzt wird.

7. Verfahren nach Anspruch 6, **dadurch gekennzeichnet, dass** aromatische Hydroxyverbindungen der Formel 8 eingesetzt werden.
Ar-OH (8),
worin
Ar Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C1-C4-Alkyl, geradkettiges oder verzweigtes C1-C4-Alkoxy, geradkettiges oder verzweigtes C1-C4-Alkoxycarbonyl, die mit Phenyl, Cyano und Halogen (z.B. F, Cl, Br) substituiert sein können und wobei weiterhin die heterocyclischen Reste mit einem ankondensierten Benzolkern verbunden sein können.

8. Verfahren nach Anspruch 6 oder 7, **dadurch gekennzeichnet, dass** weitere redoxaktive Substanzen, verwendet werden.

9. Verfahren nach einem der Ansprüche 6 bis 8, **dadurch gekennzeichnet, dass** zusätzlich Cokatalysatoren oder Hilfsmittel verwendet werden.

10. Verfahren nach Anspruch 9, **dadurch gekennzeichnet, dass** Oniumsalze, Bleiverbindungen, Polymere, Alkylhalogenide, verwendet werden.

## Claims

1. Compound of the formula (3) 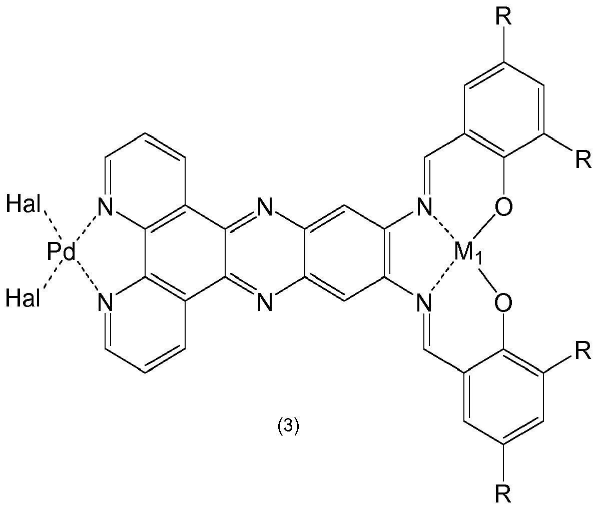 where
R is hydrogen, fluorine, chlorine, a nitro group, a C₁-C₂₂-alkyl radical or a C₆-C₂₂-aryl radical,
Hal is chloride, bromide or iodide, an alkoxide or a weakly coordinating anion and
M₁ is copper, manganese or cobalt.

2. Process for preparing the compounds of the formula 3 by hydrogenation of the nitro groups of the compound of the formula 4a to form compounds of the formula 4b, subsequent reaction with phendione of the formula 5 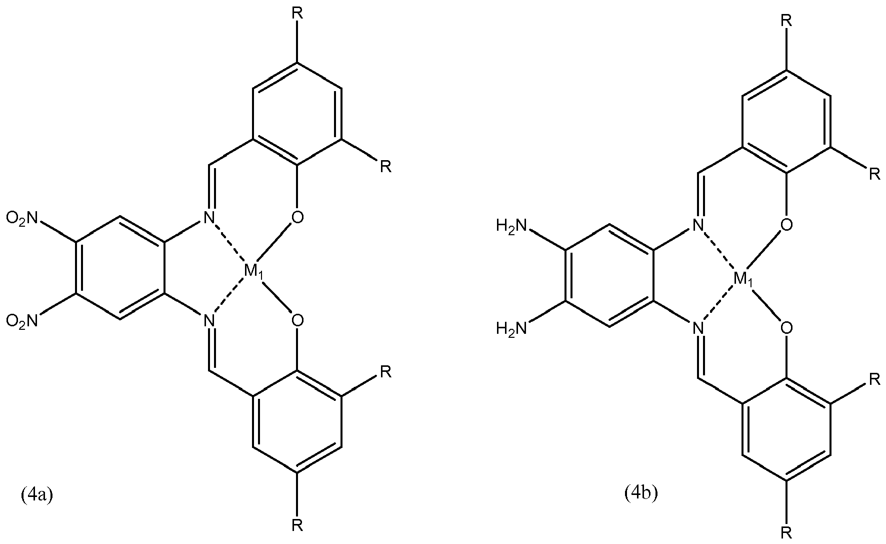 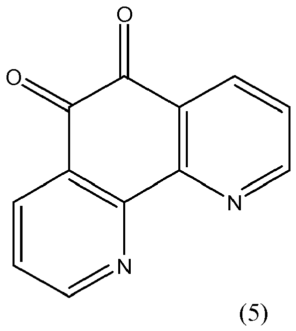 to form ligands of the formula 6 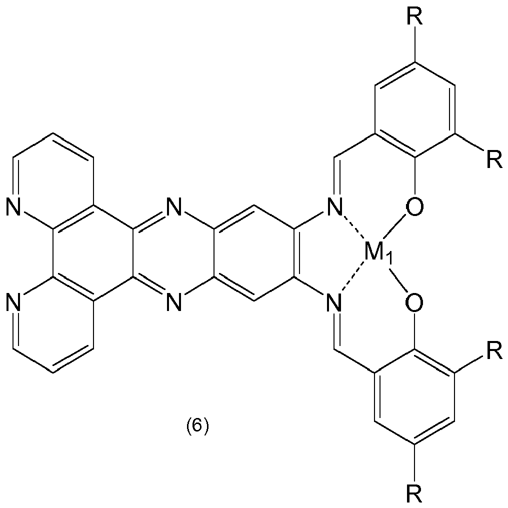 and complexation by means of a compound of the formula 7,
PdHal₂L₂ (7)
where Hal is as defined above and L is a ligand which is optionally present.

3. Compound of the general formula 4a or 4b, 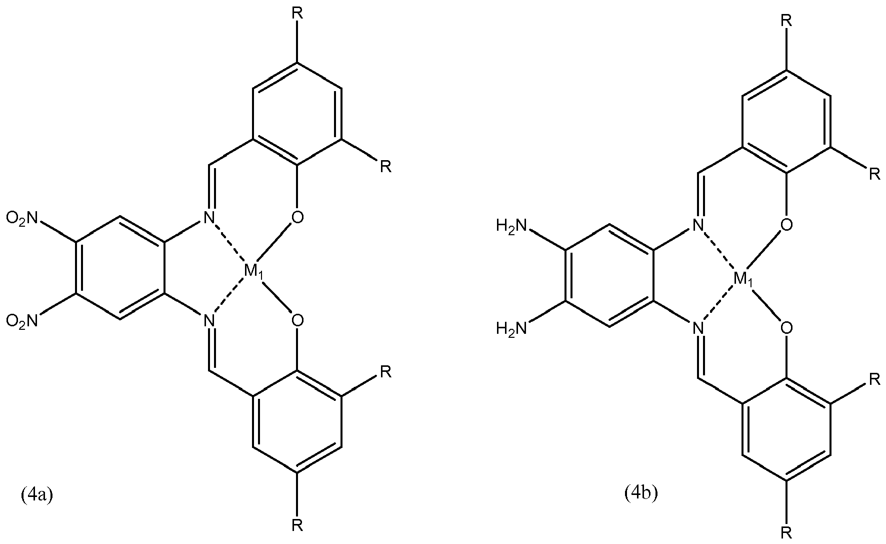 where R and M₁ are as defined above.

4. Use of one or more bimetallic compound(s) of the formula 3 according to Claim 1 as catalyst.

5. Use of one or more bimetallic compound(s) of the formula 3 according to Claim 1 for the oxidative carbonylation of aromatic hydroxy compounds to form diaryl carbonates.

6. Process for preparing diaryl carbonates, **characterized in that** an aromatic hydroxy compound is reacted with carbon monoxide and oxygen in the presence of the catalyst of the formula 3 according to Claim 1.

7. Process according to Claim 6, **characterized in that** aromatic hydroxy compounds of the formula 8
Ar-OH (8),
where
Ar is phenyl, naphthyl, anthryl, phenanthryl, indanyl, tetrahydronaphthyl or the radical of a 5- or 6-membered aromatic heterocycle having 1 or 2 heteroatoms from the group consisting of N, O and S, where these isocyclic and heterocyclic radicals may be substituted by 1 or 2 substituents such as straight-chain or branched C₁-C₄-alkyl, straight-chain or branched C₁-C₄-alkoxy, straight-chain or branched C₁-C₄-alkoxycarbonyl, each of which may be substituted by phenyl, cyano and halogen (e.g. F, Cl, Br), and the heterocyclic radicals may also be bound to a fused-on benzene ring,
are used.

8. Process according to Claim 6 or 7, **characterized in that** further redox-active substances are used.

9. Process according to any of Claims 6 to 8, **characterized in that** cocatalysts or auxiliaries are additionally used.

10. Process according to Claim 9, **characterized in that** onium salts, lead compounds, polymers, alkyl halides are used.

## Revendications

1. Composé de formule (3) 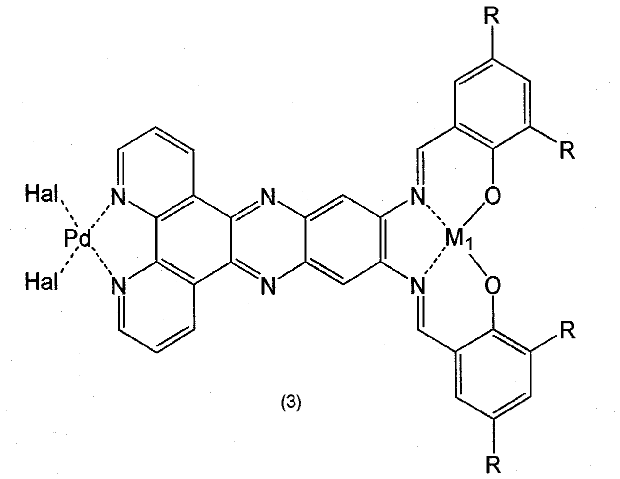 dans laquelle
R représente hydrogène, fluor, chlore, un groupe nitro, un radical C₁-C₂₂-alkyle ou un radical C₆-C₂₂-aryle
Hal représente chlorure, bromure ou iodure, un alcoolate ou un anion faiblement coordinant et
M₁ représente cuivre, manganèse ou cobalt.

2. Procédé pour la préparation des composés de formule 3 par hydrogénation des groupes nitro du composé de formule 4a en composés de formule 4b, transformation consécutive avec de la phendione de formule 5 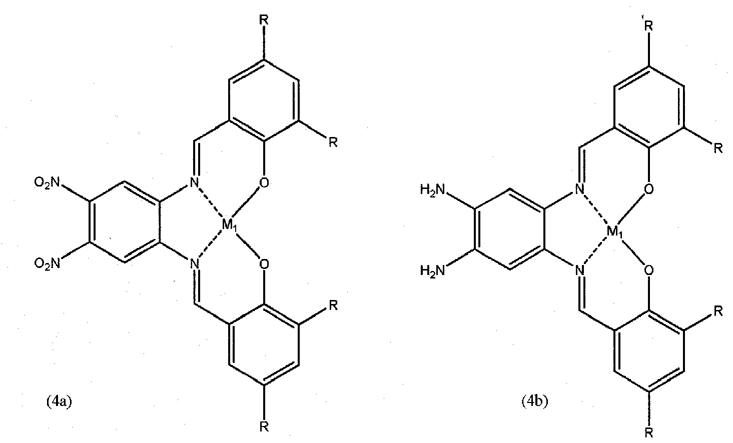 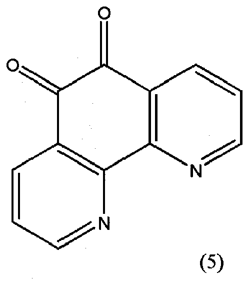 en ligands de formule 6 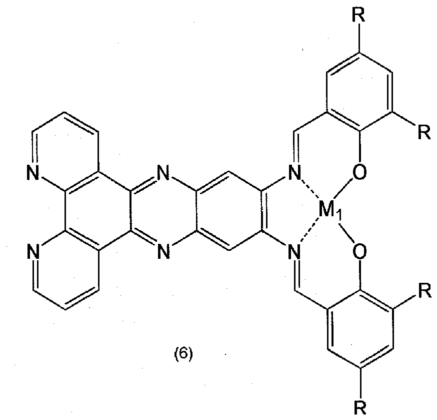 et complexation avec un composé de formule 7
PdHal₂L₂ (7)
Hal présentant la signification indiquée ci-dessus et L représentant un ligand éventuellement présent.

3. Composés des formules générales 4a et 4b 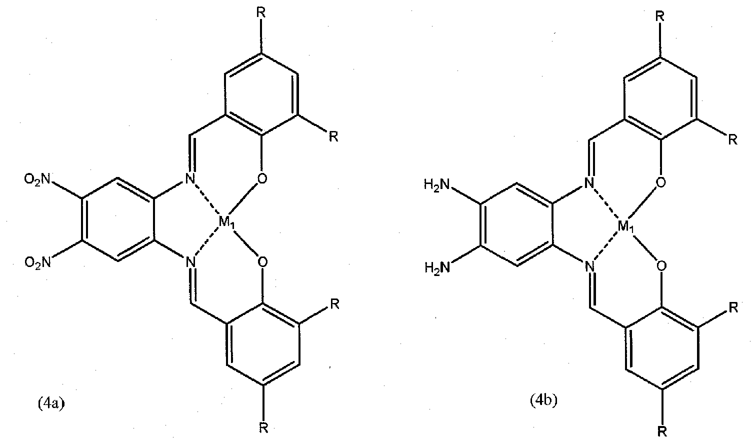 R et M₁ présentant la signification susmentionnée.

4. Utilisation d'un ou de plusieurs composés bimétalliques de formule 3 selon la revendication 1 comme catalyseur.

5. Utilisation d'un ou de plusieurs composés bimétalliques de formule 3 selon la revendication 1 pour la carbonylation oxydante de composés hydroxy aromatiques en carbonates de diaryle.

6. Procédé pour la préparation de carbonates de diaryle, **caractérisé en ce qu'**un composé hydroxy aromatique est transformé avec du monoxyde de carbone et de l'oxygène en présence du catalyseur de formule 3 selon la revendication 1.

7. Procédé selon la revendication 6, **caractérisé en ce qu'**on utilise des composés hydroxy aromatiques de formule 8
Ar-OH (8),
dans laquelle
Ar signifie phényle, naphtyle, anthryle, phénanthryle, indanyle, tétrahydronaphtyle ou le radical d'un hétérocycle aromatique de 5 ou 6 chaînons présentant 1 ou 2 hétéroatomes du groupe formé par N, 0 et S, ces radicaux isocycliques et hétérocycliques pouvant être substitués par 1 ou 2 substituants tels que C₁-C₄-alkyle linéaire ou ramifié, C₁-C₄-alcoxy linéaire ou ramifié, C₁-C₄-alcoxycarbonyle linéaire ou ramifié, qui peuvent être substitués par phényle, cyano et halogène (par exemple F, Cl, Br) et les radicaux hétérocycliques pouvant en outre être liés à un noyau benzène condensé.

8. Procédé selon la revendication 6 ou 7, **caractérisé en ce que** d'autres substances actives en redox sont utilisées.

9. Procédé selon l'une quelconque des revendications 6 à 8, **caractérisé en ce que** des cocatalyseurs ou des adjuvants sont utilisés en plus.

10. Procédé selon la revendication 9, **caractérisé en ce que** des sels d'onium, des composés du plomb, des polymères, des halogénures d'alkyle sont utilisés.
